# EUROPÄISCHE PATENTSCHRIFT

(11) **EP 3 628 225 B1**
(45) Veröffentlichungstag und Bekanntmachung des Hinweises auf die Patenterteilung: **31.03.2021**
(21) Anmeldenummer: 18196788.6
(22) Anmeldetag: 26.09.2018
(51) Int. Cl.: A61B 6/00, A61B 34/30

(54) **VERFAHREN ZUR AUFNAHME VON BILDDATEN UND MEDIZINISCHES BILDGEBUNGSSYSTEM**
METHOD FOR RECORDING IMAGE DATA AND MEDICAL IMAGING SYSTEM
PROCÉDÉ D'ENREGISTREMENT DE DONNÉES D'IMAGE ET SYSTÈME D'IMAGERIE MÉDICALE

(43) Veröffentlichungstag der Anmeldung: 01.04.2020
(73) Patentinhaber: Siemens Healthcare GmbH, 91052 Erlangen (DE)
(72) Erfinder: Fischer, Peter, 91466 Gerhardshofen (DE); Mewes, Philip, 90419 Nürnberg (DE); Müller, Gunter, 90562 Heroldsberg (DE)

(56) Entgegenhaltungen:
- DE-A1-102007 007 386
- DE-A1-102008 022 924
- DE-A1-102014 224 122
- US-A1- 2016 249 990
- US-A1- 2017 202 629

## Beschreibung

Die Erfindung betrifft ein Verfahren zur Aufnahme von Bilddaten eines sich insbesondere zyklisch bewegenden Untersuchungsobjekts gemäß dem Patentanspruch 1 sowie ein Medizinisches Bildgebungssystem zur Durchführung eines derartigen Verfahrens gemäß dem Patentanspruch 8.

Bei der medizinischen Bildgebung beweglicher anatomischer Strukturen eines Patienten kommt es, z.B. durch Atmung, Herzschlag oder durch Manipulation am Körper bedingt, zu Inkonsistenzen in der aufgenommenen Bildinformation. Dies ist insbesondere bei zeitversetzter aufeinander aufbauender Bildgebung in Verbindung mit fehlender Information über die Patientenposition zu den beiden Aufnahmezeitpunkten problematisch. Für den Fall einer 2d-2d-3d Registrierung ist eine Patientenbewegung zwischen der ersten 2d(1)-Aufnahme und der zweiten 2d(2)-Aufnahme besonders kritisch. Wenn sich der Patient zwischen der ersten 2d(1)-Aufnahme und der zweiten 2d(2)-Aufnahme bewegt, kommt es z.B. bei der Registrierung beider Aufnahmen zu einem 3d Volumen zu einer artefaktbehafteten Mittelung zwischen 2d(1)-Aufnahme und 2d(2)-Aufnahme oder einer Fokussierung bzw. Optimierung auf entweder die erste 2d(1)-Aufnahme oder die zweite 2d(2)-Aufnahme.

Basierend auf der Bildgebung besteht dann für eine nachfolgende Intervention die analoge Problematik, dass durch etwaige Bewegungen in der Anatomie Zielpositionen obsolet werden. Z.B. im Fall eines Schnittes in Gewebe oder eines Bohren in Knochen ist die durch Bildgebung festgelegte Zielposition eines Schnitts oder eines Bohrlochs aufgrund der Bewegung in der Realität nicht konstant und somit läuft der Chirurg Gefahr, die eigentliche Zielposition zu verfehlen. Dies kann neben falschen Diagnosen zu falsch gesetzten Schnitten oder Bohrungen sowie zu erheblichen ungewollten Verletzungen des Patienten führen.

In bekannten Fällen wird zur Vermeidung solcher Probleme z.B. die Bewegung des Patienten unterbunden (Atmung anhalten, Patientenanatomie fixieren) oder der Patient mittels Navigationsgeräten getrackt bzw. seine Bewegung gemessen (z.B. Atemgürtel) und dann die Bewegung entsprechend in die Aufnahmeplanung bzw. Auswertung einbezogen.

Aus der DE 10 2008 022924 A1 ist eine Vorrichtung mit einem Bildgebungsgerät und einem an einem Roboterarm angeordneten medizinischen Instrument, z.B. einer Biopsienadel, bekannt. Das Instrument weist zusätzlich einen elektromagnetischen Sensor zur Positionserfassung auf, dessen Signal mit den Bewegungen des Roboter synchronisiert wird. Aus der DE 10 2007 007386 A1 ist ein Verfahren zur Kompensation von Bilddaten mit gleichzeitig akquirierten Bewegungsdaten bekannt. Hier werden mittels eines an einem Roboterarm angeordneten Ultraschalltransducers Bewegungen des Herzens aufgenommen und damit Bilddaten eines Röntgengeräts bewegungskompensiert.
Es ist Aufgabe der vorliegenden Erfindung, ein Verfahren zur Aufnahme von Bilddaten eines sich insbesondere zyklisch bewegenden Untersuchungsobjekts bereitzustellen, welches eine möglichst bewegungsunabhängige Bildgebung ermöglicht; des Weiteren ist es Aufgabe der Erfindung, ein für die Durchführung des Verfahrens geeignetes Röntgengerät bereitzustellen. Die Aufgabe wird erfindungsgemäß gelöst durch ein Verfahren zur Aufnahme von Bilddaten eines sich insbesondere zyklisch bewegenden Untersuchungsobjekts gemäß dem Patentanspruch 1 und von einem medizinischen Bildgebungssystem gemäß dem Patentanspruch 8. Vorteilhafte Ausgestaltungen der Erfindung sind jeweils Gegenstand der zugehörigen Unteransprüche.

Das erfindungsgemäße Verfahren ist zur Aufnahme von Bilddaten eines sich insbesondere zyklisch bewegenden Untersuchungsobjekts eines Patienten mittels eines medizinischen Bildgebungssystems, insbesondere mit einer Röntgenquelle und einem Röntgendetektor, ausgebildet, wobei ein robotisches Gerät mit einer kinematischen Kette von bewegbaren Komponenten mit dem Patienten taktil in Verbindung steht, indem ein Endeffektor auf der Oberfläche des Patienten aufliegt, und wobei die taktile Verbindung zumindest über die Dauer des Verfahrens mittels einer Kraft- und/oder Momentenregelung durch Mitbewegen der bewegbaren Komponenten aufrechterhalten wird, und wobei währenddessen folgende Schritte erfolgen: Erfassung von Messwerten durch eine Kraft- und/oder Momenten- und/oder Positionssensorik des robotischen Gerätes, Auswertung der Messwerte und Weitergabe an das medizinische Bildgebungssystem, wobei die ausgewerteten Messwerte insbesondere Informationen zur Bewegung und/oder Position des Untersuchungsobjekts aufweisen, und Aufnahme von Bilddaten des, insbesondere durchstrahlten, Untersuchungsobjekts, insbesondere bei Bestrahlung des Untersuchungsobjekt durch die Strahlungsquelle und Aufnahme durch den Röntgendetektor, wobei die ausgewerteten Messwerte für eine Ansteuerung des Bildgebungssystems verwendet werden. Zyklische Bewegungen, z.B. Atembewegungen oder Herzbewegungen, oder andere Bewegungen, z.B. durch einen Eingriff induzierte Bewegungen, eines Patienten beeinflussen das Untersuchungsobjekt, also ein beliebiges Organ (z.B. Leber) des Patienten, mit und führen auch dort zu (z.B. zyklischen) Bewegungen, welche die Bilddaten z.B. bei einer Röntgenaufnahme beeinflussen.

Bei Eingriffen, bei denen standardmäßig ein robotisches Gerät mit einer kinematischen Kette von bewegbaren Komponenten vorhanden ist und eingesetzt wird, kann dieses gemäß der Erfindung nicht nur für den Eingriff selbst sondern auch für die Bestimmung von Informationen über die (zyklischen) Bewegungen eines Untersuchungsobjekts und damit auch für eine Ansteuerung des Bildgebungssystems verwendet werden. Es wird also gezielt eine bereits vorhandene Ressource, das robotische Gerät, welches eine taktile Verbindung zu dem Patienten aufweist, genutzt, so dass keine zusätzlichen Maßnahmen oder Gerate zur Messung der Bewegung oder Position des Untersuchungsobjekts vorgesehen werden müssen. Insgesamt ist es möglich, aber nicht nötig, dass das robotische Gerät räumlich zu dem medizinischen Bildgebungssystem registriert ist. Das Verfahren ist insbesondere für eine Röntgenbildgebung geeignet, kann aber auch bei anderen Bildgebungsverfahren wie der Magnetresonanztomographie oder der Ultraschallbildgebung verwendet werden.

Nach einer Ausgestaltung der Erfindung werden die Bestrahlung des Untersuchungsobjekts und die Aufnahme der Bilddaten getriggert in Abhängigkeit von den ausgewerteten Messwerten, insbesondere von Bewegungs- und/oder Positionsdaten des Untersuchungsobjekts, gesteuert. Hierbei wird das robotische Gerät auf einfache Weise für eine gezielte Triggerung bei der Bildgebung genutzt. Dies ist besonders vorteilhaft, da durch das robotische Gerät mittels des taktilen Kontakts eine besonders genaue Bestimmung der Bewegungen und/oder Positionen möglich ist. Es kann in vorteilhafter Weise zumindest eine Projektionsaufnahme des Untersuchungsobjekts zu einem vorbestimmten Zeitpunkt und/oder einer vorbestimmten Phase der, insbesondere zyklischen, Bewegung des Untersuchungsobjekts aufgenommen werden. Bei zyklischen Bewegungen kann z.B. eine gezielte Atemtriggerung (respiratory gating) oder Herzschlagtriggerung (cardiac gating) durchgeführt werden, wobei ein vorbestimmter Zeitpunkt oder eine vorbestimmte Phase ausgenutzt wird. Bei anderen (nicht-zyklischen) Bewegungen kann eine Ruhephase für die Bildgebung genutzt werden.

Nach einer weiteren Ausgestaltung der Erfindung werden zumindest zwei Projektionsaufnahmen des Untersuchungsobjekts zum selben Zeitpunkt und/oder derselben Phase der insbesondere zyklischen Bewegung des Untersuchungsobjekts aufgenommen. Die Aufnahmen in vergleichbaren Phasen oder zu gleichen Zeitpunkten einer zyklischen Bewegung erleichtert die Vergleichbarkeit der Aufnahmen, so dass eine nachträgliche Bewegungskompensation vereinfacht wird oder vollständig ausbleiben kann.

Insbesondere bei Registrierungen wie der 2D-2D-3D-Registrierung kann das Verfahren zum Einsatz kommen, wobei die beiden Projektionsaufnahmen aus verschiedenen Projektionsrichtungen (Angulationen eines Aufnahmesystems des medizinischen Bildgebungssystems) aufgenommen und für die 2D-2D-3D-Registrierung mit einem zuvor aufgenommenen oder aus einer anderen Modalität zur Verfügung gestellten Volumenbild verwendet werden. Hierdurch wird die Registrierung vereinfacht und kann mit hoher Genauigkeit durchgeführt werden. Dies erleichtert weitere Diagnosestellungen und Bildverarbeitungen und verbessert damit die Patientensicherheit und -versorgung.

Nach einer weiteren Ausgestaltung der Erfindung wird eine Bearbeitung der aufgenommenen Bilddaten unter Verwendung der ausgewerteten Messwerte durchgeführt. Durch die ausgewerteten Messwerte wird eine Bearbeitung der Aufnahmen erleichtert. So können dadurch auf einfache Weise Bewegungen nachvollzogen und entsprechend korrigiert werden.

Erfindungsgemäß werden die Messwerte von einer Kraft- und/oder Momenten- und/oder Positionssensorik des robotischen Geräts erfasst. Übliche robotische Geräte mit einer kinematischen Kette von bewegbaren Komponenten besitzen z.B. im Bereich ihrer Gelenke derartige Sensoren, welche für die bekannte Positionierung der Komponenten verwendet werden.

Ein Verfahren zur Aufnahme von Bilddaten eines sich, insbesondere zyklisch, bewegenden Untersuchungsobjekts eines Patienten mittels eines medizinischen Bildgebungssystems, insbesondere mit einer Röntgenquelle und einem Röntgendetektor, wobei ein robotisches Gerät mit einer kinematischen Kette von bewegbaren Komponenten mit dem Patienten taktil in Verbindung steht, und wobei die taktile Verbindung zumindest über einen vorgegebenen Zeitraum insbesondere durch Mitbewegen der bewegbaren Komponenten aufrechterhalten wird, umfasst währenddessen die folgenden Schritte: Aufnahme von Bilddaten des, insbesondere durchstrahlten, Untersuchungsobjekts, insbesondere bei Bestrahlung des Untersuchungsobjekt durch die Strahlungsquelle und Aufnahme durch den Röntgendetektor, und Erfassung von Messwerten durch Sensoren des robotischen Gerätes, wobei anschließend eine Auswertung der Messwerte und Weitergabe an das medizinische Bildgebungssystem erfolgt, wobei die ausgewerteten Messwerte insbesondere Informationen zur Bewegung und/oder Position des Untersuchungsobjekts aufweisen, und wobei eine Bearbeitung der aufgenommenen Bilddaten unter Verwendung der ausgewerteten Messwerte durchgeführt wird. Hier werden die aufgenommenen Messwerte (bzw. Information über die Bewegungen und oder Positionen des Untersuchungsobjekts) also in der weiteren Bildverarbeitung berücksichtigt, um z.B. Bewegungskorrekturen durchzuführen, Inkonsistenzen zu reduzieren oder zu vermeiden und damit die Diagnosemöglichkeiten zu verbessern. So kann z.B. in dem Fall der 2D-2D-3D-Registrierung eine einfache und effektive Korrektur der Bewegung durchgeführt werden und somit ein hochqualitatives Ergebnis erzielt werden.

Erfindungsgemäß ist ein medizinisches Bildgebungssystem zum Erfassen von Bilddaten eines sich zyklisch bewegenden Untersuchungsobjekts eines Patienten vorgesehen, aufweisend eine Bildgebungseinheit zur Aufnahme von Bilddaten des Untersuchungsobjekts, insbesondere eine Strahlungsquelle zum Aussenden einer Röntgenstrahlung und einen Röntgendetektor zur Aufnahme von Bilddaten des durchstrahlten Untersuchungsobjekts, eine Steuerungseinheit zur getriggerten Ansteuerung der (Strahlungsausgabe und der) Aufnahme von Bilddaten, ein robotisches Gerät mit einer kinematischen Kette von bewegbaren Komponenten, aufweisend eine Kraft- und/oder Momenten- und/oder Positionssensorik zur Erfassung von Messwerten, welche insbesondere Informationen zur Bewegung und/oder Position des Untersuchungsobjekts aufweisen, wobei das robotische Gerät mit dem Patienten taktil in Verbindung steht und dazu ausgebildet ist, die taktile Verbindung für die Dauer des Verfahrens mittels der Kraft- und/oder Momentenregelung durch Mitbewegen der bewegbaren Komponenten aufrechtzuerhalten, eine Auswerteeinheit zur Auswertung der Messwerte und eine Kommunikationsverbindung zur Weitergabe der Messwerte, wobei das medizinische Bildgebungssystem für eine Verwendung der ausgewerteten Messwerte für eine Ansteuerung des Bildgebungssystems ausgebildet ist.

Vorteilhafterweise weist das robotische Gerät an einem Ende einen Endeffektor auf, welcher mit dem Patienten taktil in Kontakt steht, also auf der Oberfläche des Patienten oder des Untersuchungsobjekts (Organs) oder eines Knochens (z.B. Wirbelsäule, Femur) des Patienten aufliegt oder mit ihr verbunden ist. So kann insbesondere bei interventionellen Eingriffen der Endeffektor eine gezahnte Hülse aufweisen und mit einem Knochen des Patienten (z.B. bei der Wirbelsäulenchirurgie mit einem Wirbelkörper bzw. Pedikel oder Lamina) in Kontakt stehen.

In vorteilhafter Weise ist die Steuerungseinheit derart ausgebildet, dass sie die Bestrahlung des Untersuchungsobjekts und die Aufnahme der Bilddaten getriggert in Abhängigkeit von den ausgewerteten Messwerten steuert.

Nach einer Ausgestaltung weist das medizinische Bildgebungssystem eine Bildbearbeitungseinheit zur Bearbeitung der Bilddaten unter Verwendung der ausgewerteten Messwerte auf.

Außerdem weist das medizinische Bildgebungssystem nach einer weiteren Ausgestaltung eine Registrierungseinheit zur Durchführung einer 2D-2D-3D-Registrierung zwischen zwei aus verschiedenen Projektionsrichtungen zum selben Zeitpunkt und/oder derselben Phase der zyklischen Bewegung des Untersuchungsobjekts aufgenommenen (2D-)Projektionsaufnahmen und einem 3D-Volumenbild des Untersuchungsobjekts auf.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Schrittabfolge eines Verfahrens zur Aufnahme von Bilddaten;
- FIG 2: eine weitere Schrittabfolge eines Verfahrens zur Aufnahme von Bilddaten; und
- FIG 3: ein erfindungsgemäßes medizinisches Bildgebungssystem zur Durchführung des Verfahrens.

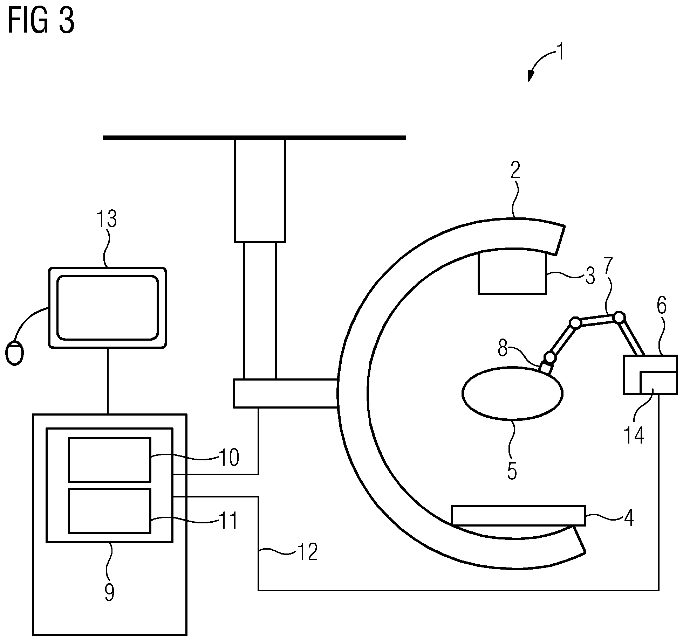

In der FIG 3 ist ein medizinisches Bildgebungssystem 1 mit einem robotischen Gerät 6 gezeigt. Das medizinische Bildgebungssystem 1 weist z.B. als Aufnahmesystem einen C-Bogen 2 mit einer Röntgenquelle 3 und einem Röntgendetektor 4 auf, welche zur Bestrahlung eines Untersuchungsobjekts 5 (z.B. Organ oder Körperteil) eines Patienten und Aufnahme von Projektionsbildern des durchstrahlten Untersuchungsobjekts 5 ausgebildet sind. Der C-Bogen 2 kann dabei derart verstellbar bzw. bewegbar ausgebildet sein, dass er Projektionsbilder aus verschiedenen Projektionsrichtungen (Angulationen) aufnehmen kann. Das medizinische Bildgebungssystem 1 wird mittels einer Steuerungseinheit 9 angesteuert und weist außerdem eine Recheneinheit 10 sowie eine Bildverarbeitungseinheit 11 mit einer Software zur Verarbeitung aufgenommener Bilddaten auf. Außerdem steht das medizinische Bildgebungssystem 1 mit einem robotischen Gerät 6 in Kommunikationsverbindung 12. Die Kommunikationsverbindung 12 kann kabelgebunden oder kabellos vorgesehen sein.

Das robotische Gerät 6 weist eine kinematische Kette aus bewegbaren Komponenten 7 (z.B. Armen und Gelenken) auf und an ihrem freien Ende ist ein Endeffektor 8 angeordnet, welcher mit dem Untersuchungsobjekt 5 oder einem anderen Teil des Patienten in Kontakt stehen kann. Das robotische Gerät 6 kann z.B. von einem Roboter mit Knickarmen wie einem Leichtbauroboter oder einem 6- bzw. 7-Achs-Knickarmroboter gebildet werden. Bei dem Endeffektor kann es sich z.B. um eine gezahnte Hülse handeln, welche z.B. mit einem Wirbelkörper des Patienten für einen Eingriff in Kontakt ist. Durch die Zahnung ist es möglich, ein Verrutschen oder Abreißen des Kontakts zu verhindern.

Das robotische Gerät kann bei Bewegungen oder Positionsveränderungen des Patienten so angesteuert werden, dass es sich mit dem Körperteil, mit dem es in Kontakt steht, derart mitbewegt, dass der Kontakt bestehen bleibt. Es kann sogar ein entsprechender Anpressdruck aufrechterhalten werden. Dies kann z.B. mittels einer Kraft- und/oder Momentenregelung durchgeführt werden. Hierfür weisen die bewegbaren Komponenten 7 des robotischen Geräts eine Kraft- und/oder Momenten- und/oder Positionssensorik auf, also zumindest einen derartigen Sensor pro bewegbare Komponente. Beispiele für solche robotischen Geräte sind z.B. aus der Veröffentlichungsschrift DE 102014224122 A1 oder der nachveröffentlichten Anmeldung EP 17191039 bekannt.

Die Kraft- und/oder Momenten- und/oder Positionssensoren des robotischen Gerätes erfassen Messwerte, welche entweder direkt oder indirekt Informationen oder Daten zur Position oder Bewegung des Untersuchungsobjekts oder des Körperteils, mit welchem sie verbunden sind, oder des Patienten anzeigen. So sind derartige Kraft- oder Drehmomentsensoren bekannt und besonders gut geeignet, Kräfte und/oder Drehmomente an der kinematischen Kette von robotischen Geräten zu bestimmen. Dabei werden die einwirkenden Kräfte entweder direkt gemessen oder es werden Werte gemessen, aus welchen auf einfache Weise die einwirkende Kraft bestimmt bzw. berechnet werden kann. Im Allgemeinen werden die Messwerte also ausgewertet (verarbeitet oder umgerechnet), um die Position oder Bewegung zu ergeben. Dies kann jedoch sehr schnell mittels einer Auswerteeinheit 14 (z.B. eine Steuerungseinheit und/oder Recheneinheit des robotischen Geräts) durchgeführt werden.

Mittels eines derartigen medizinischen Bildgebungssystems 1 in Kombination mit einem robotischen Gerät 6 kann ein Verfahren wie folgt durchgeführt werden.

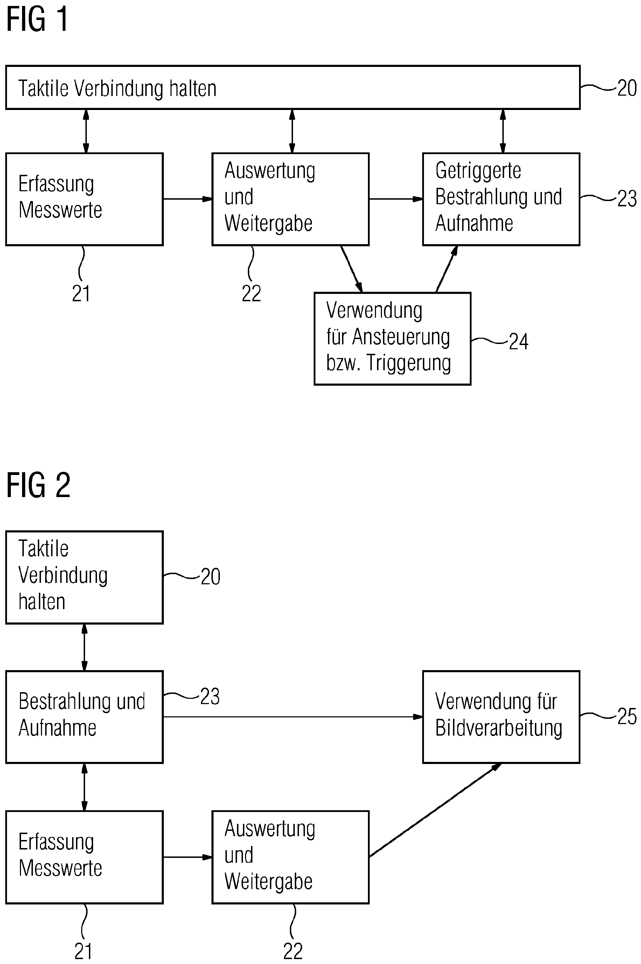

In einer ersten Variante - gezeigt in FIG 1 - ist ein Verfahren gezeigt, bei welchem ausgewertete Messwerte mit Informationen zu Position und/oder Bewegung des Patienten oder des Untersuchungsobjekts zur Ansteuerung der Bildgebung des medizinischen Bildgebungssystems verwendet werden. Konkret wird mit den Informationen eine Triggerung der Bildgebung zu gewünschten Zeitpunkten bzw. Phasen von (zyklischen) Bewegungen des Patienten oder Untersuchungsobjekts durchgeführt, während gleichzeitig das robotische Gerät mit dem Patienten in Verbindung ist.

In einem ersten Schritt 20 wird von dem robotischen Gerät 6, welches mit dem sich (z.B. zyklisch) bewegenden Patienten taktil in Verbindung steht, die taktile Verbindung zumindest über einen vorgegebenen Zeitraum durch Mitbewegen der bewegbaren Komponenten aufrechterhalten. So kann z.B. in einer nicht-beanspruchten Variante des Verfahrens ein robotisches Gerät 6, welches für einen interventionellen Eingriff an Knochengewebe eines Patienten (z.B. Wirbelsäule) mittels einer gezahnten Hülse als Endeffektor mit dem Knochen in Verbindung steht, mittels Kraft- und/oder Momenten- und/oder Positionssensorik mit der Position des Knochens mitgehen und dabei auf dem Knochen stabilisiert werden. Der vorgegebene Zeitraum, währenddessen die taktile Verbindung aufrechterhalten wird, kann dabei z.B. der Dauer des Verfahrens entsprechen, oder einem beliebig von einem Nutzer des medizinischen Bildgebungssystems vorgegebenen Zeitraum oder kontinuierlich bis zu einem wählbaren Abbruchkriterium andauern. Die folgenden zweiten bis fünften Schritte erfolgen während die taktile Verbindung aufrechterhalten wird. Die taktile Verbindung kann mit dem Untersuchungsobjekt oder einem anderen Körperteil des Patienten bestehen.

In einem zweiten Schritt 21 werden von dem robotischen Gerät 6 bzw. dessen Sensorik Messwerte erfasst und diese Messwerte in einem dritten Schritt 22 ausgewertet und an das medizinische Bildgebungssystem weitergegeben (nicht zwingend in dieser Reihenfolge). Die ausgewerteten Messwerte enthalten dabei insbesondere Informationen zur Bewegung und/oder Position des Untersuchungsobjekts bzw. des Patienten. Konkret können die Messwerte direkt oder indirekt z.B. die wirkende Kraft auf die bewegbaren Komponenten des robotische Gerät in verschiedenen Richtungen oder die Position des der bewegbaren Komponenten des robotischen Geräts bei nachgiebiger Regelung enthalten. Die Auswertung kann durch eine Auswerteeinheit 14 des robotischen Geräts oder auch von dem medizinischen Bildgebungssystem durchgeführt werden. Sie kann z.B. derart sein, dass die genauen Positionen des Patienten, eines Körperteils des Patienten oder des Untersuchungsobjekts während einer (zyklischen) Bewegung bzw. die (relative oder absolute) Bewegung ermittelt wird.

In einem vierten Schritt 23 werden die Informationen bzw. Messdaten bzw. ausgewerteten Messdaten (insbesondere Bewegungs- und/oder Positionsdaten des Untersuchungsobjekts) dann von dem medizinischen Bildgebungssystem (z.B. Systemssteuerung und/oder Softwareapplikationen) dafür verwendet, um in einem fünften Schritt 24 eine oder mehrere getriggerte Röntgenaufnahmen (also Bestrahlung durch die Röntgenquelle und Erfassung der vom Untersuchungsobjekt beeinflussten Strahlung durch den Röntgendetektor und Umwandlung in Bilddaten) durchzuführen. Im einfachsten Fall wird also z.B. eine Atemtriggerung oder Herzschlagtriggerung durchgeführt, bei welcher zu gleichen Zeitpunkten in mehreren aufeinander folgenden Phasen der Atmung oder des Herzschlags je eine Aufnahme durchgeführt wird, um die mehreren resultierenden Projektionsaufnahmen miteinander vergleichen zu können.

So kann z.B. im Rahmen einer 2D-2D-3D-Registrierung, bei welcher ein zuvor aufgenommenes Volumenbild mit zwei Projektionsaufnahmen aus verschiedenen Projektionsrichtungen registriert wird, ein solches Verfahren verwendet, um die beiden Projektionsaufnahmen zu gleichen Zeitpunkten aufeinander folgender Atemzyklen (oder Herzzyklen) aus verschiedenen Projektionsrichtungen des Aufnahmesystems getriggert aufzunehmen. Anschließend kann eine besonders genaue und präzise 2D-2D-3D-Registrierung durchgeführt werden. Dafür ist es möglich, aber nicht nötig, dass das robotische Gerät räumlich zum medizinischen Bildgebungssystem registriert ist. Zusätzlich kann auch die Information über die Bewegungen bzw. Positionen des Untersuchungsobjekts in eine spätere Bildverarbeitung einbezogen werden, um Bewegungskompensationen durchzuführen und/oder Inkonsistenzen zu vermeiden bzw. reduzieren. Z.B. können Information über die Position in die Registrierung einfließen lassen. So ist z.B. die Atembewegung der Wirbelsäule hauptsachlich in anterior-posterior Richtung, die vom robotischen Gerät gemessen wird und als relative Transformation in der Registrierung berücksichtigt werden kann.

So kann mittels des Verfahrens ein robotisches Gerät mit bewegbaren Komponenten, welches zur geführten Intervention in Kontakt mit den anatomischen Strukturen eines Patienten steht gleichzeitig als integriertes Gating Triggering Device benutzt werden.

Außerdem - gezeigt in FIG 2 - ist ein Verfahren gezeigt, bei welchem lediglich eine Bearbeitung der aufgenommenen Bilddaten unter Verwendung ausgewerteter Messwerte durchgeführt wird. Hier werden gleichzeitig während in dem ersten Schritt 20 die taktile Verbindung aufrechterhalten wird, im vierten Schritt 23 eine oder mehrere Röntgenaufnahmen durchgeführt und im zweiten Schritt 21 entsprechende Messwerte von der Sensorik des robotischen Geräts erfasst. Die ausgewerteten Messwerte enthalten ebenfalls Informationen zu Position und/oder Bewegung des Untersuchungsobjekts. Diese werden an das medizinische Bildgebungssystem weitergegeben (oder erst dort ausgewertet) und anschließend in einem sechsten Schritt 25 für die Bildverarbeitung weiterverwendet. Z.B. kann die Information über die Bewegungen bzw. Positionen des Untersuchungsobjekts in die Bildverarbeitung einbezogen werden, um Bewegungskompensationen durchzuführen und/oder Inkonsistenzen zu vermeiden bzw. reduzieren.

Außerdem kann mittels des erfindungsgemäßen medizinischen Bildgebungssystems auch ein Interventionsschritt abhängig von der aktuellen Position oder Bewegung des Untersuchungsobjekts freigegeben bzw. initiiert werden.

Bei Eingriffen, bei denen standardmäßig ein robotisches Gerät mit einer kinematischen Kette von bewegbaren Komponenten vorhanden ist und eingesetzt wird, kann nicht nur für den Eingriff selbst sondern auch für die Bestimmung von Informationen über die (zyklischen) Bewegungen eines Untersuchungsobjekts und damit auch für eine Ansteuerung des medizinischen Bildgebungssystems (z.B. Atem gating oder Herz gating) verwendet werden.

Die Erfindung lässt sich in folgender Weise kurz zusammenfassen: Vorgesehen ist ein Verfahren zur Aufnahme von Bilddaten eines sich, insbesondere zyklisch, bewegenden Untersuchungsobjekts eines Patienten mittels eines medizinischen Bildgebungssystems mit einer Röntgenquelle und einem Röntgendetektor, wobei ein robotisches Gerät mit einer kinematischen Kette von bewegbaren Komponenten mit dem Patienten taktil in Verbindung steht, indem ein Endeffektor auf der Oberfläche des Patienten aufliegt, und wobei die taktile Verbindung zumindest für die Dauer des Verfahrens mittels einer Kraft-und/oder Momentenregelung durch Mitbewegen der bewegbaren Komponenten aufrechterhalten wird, und wobei währenddessen folgende Schritte erfolgen: Erfassung von Messwerten durch Kraft- und/oder Momenten-und/oder Positionssensorik des robotischen Gerätes, Auswertung der Messwerte und Weitergabe an das medizinische Bildgebungssystem, wobei die ausgewerteten Messwerte insbesondere Informationen zur Bewegung und/oder Position des Untersuchungsobjekts aufweisen, und Bestrahlung des Untersuchungsobjekt durch die Strahlungsquelle und Aufnahme von Bilddaten des durchstrahlten Untersuchungsobjekts durch den Röntgendetektor, und wobei die ausgewerteten Messwerte für eine Ansteuerung des Bildgebungssystems verwendet werden.

## Patentansprüche

1. Verfahren zur Aufnahme von Bilddaten eines sich, insbesondere zyklisch, bewegenden Untersuchungsobjekts (5) eines Patienten mittels eines medizinischen Bildgebungssystems (1), wobei ein robotisches Gerät (6) mit einer kinematischen Kette von bewegbaren Komponenten (7) mit dem Patienten taktil in Verbindung steht, indem ein Endeffektor auf der Oberfläche des Patienten aufliegt, und wobei die taktile Verbindung zumindest für die Dauer des Verfahrens mittels einer Kraft- und/oder Momentenregelung durch Mitbewegen der bewegbaren Komponenten (7) aufrechterhalten wird, und wobei währenddessen folgende Schritte erfolgen:
• Erfassung von Messwerten durch Kraft- und/oder Momenten- und/oder Positionssensorik des robotischen Gerätes (6),
• Auswertung der Messwerte und Weitergabe an das medizinische Bildgebungssystem (1), wobei die ausgewerteten Messwerte insbesondere Informationen zur Bewegung und/oder Position des Untersuchungsobjekts (5) aufweisen, und
• Aufnahme von Bilddaten des Untersuchungsobjekts (5) durch das Bildgebungssystem (1),
wobei die ausgewerteten Messwerte für eine Ansteuerung des Bildgebungssystems (1) verwendet werden.

2. Verfahren nach Anspruch 1, wobei das medizinische Bildgebungssystem (1) eine Röntgenquelle (3) und einen Röntgendetektor (4) aufweist und die Aufnahme von Bilddaten eine Bestrahlung des Untersuchungsobjekts (5) durch die Röntgenquelle (3) und eine Aufnahme des durchstrahlten Untersuchungsobjekts (5) durch den Röntgendetektor (4) umfasst.

3. Verfahren nach Anspruch 2, wobei die Bestrahlung des Untersuchungsobjekts (5) und die Aufnahme der Bilddaten getriggert in Abhängigkeit von den ausgewerteten Messwerten, insbesondere von Bewegungs- und/oder Positionsdaten des Untersuchungsobjekts (5), gesteuert werden.

4. Verfahren nach Anspruch 2 oder 3, wobei zumindest eine Projektionsaufnahme des Untersuchungsobjekts (5) zu einem vorbestimmten Zeitpunkt und/oder einer vorbestimmten Phase der insbesondere zyklischen Bewegung des Untersuchungsobjekts (5) aufgenommen wird.

5. Verfahren nach Anspruch 2 oder 3, wobei zumindest zwei Projektionsaufnahmen des Untersuchungsobjekts (5) zum selben Zeitpunkt und/oder derselben Phase der insbesondere zyklischen Bewegung des Untersuchungsobjekts (5) aufgenommen werden.

6. Verfahren nach Anspruch 5, wobei die beiden Projektionsaufnahmen aus verschiedenen Projektionsrichtungen aufgenommen und für eine 2D-2D-3D-Registrierung verwendet werden.

7. Verfahren nach einem der vorangehenden Ansprüche, wobei eine Bearbeitung der aufgenommenen Bilddaten unter Verwendung der ausgewerteten Messwerte durchgeführt wird.

8. Medizinisches Bildgebungssystem (1) zum Erfassen von Bilddaten eines sich zyklisch bewegenden Untersuchungsobjekts (5) eines Patienten, ausgebildet zur Durchführung eines Verfahrens zur Aufnahme von Bilddaten des sich zyklisch bewegenden Untersuchungsobjekts, aufweisend
• eine Bildgebungseinheit (3; 4) zur Aufnahme von Bilddaten des Untersuchungsobjekts (5),
• eine Steuerungseinheit (9) zur getriggerten Ansteuerung der Aufnahme von Bilddaten,
• ein robotisches Gerät (6) mit einer kinematischen Kette von bewegbaren Komponenten (7), aufweisend eine Kraft- und/oder Momenten- und/oder Positionssensorik zur Erfassung von Messwerten, welche insbesondere Informationen zur Bewegung und/oder Position des Untersuchungsobjekts aufweisen, wobei das robotische Gerät (6) mit dem Patienten taktil in Verbindung steht und dazu ausgebildet ist, die taktile Verbindung für die Dauer des Verfahrens mittels der Kraft- und/oder Momentenregelung durch Mitbewegen der bewegbaren Komponenten (7) aufrechtzuerhalten,
• eine Auswerteeinheit (14) zur Auswertung der Messwerte und
• eine Kommunikationsverbindung (12) zur Weitergabe der Messwerte,
wobei das medizinische Bildgebungssystem (1) für eine Verwendung der ausgewerteten Messwerte für eine Ansteuerung des Bildgebungssystems (1) ausgebildet ist.

9. Medizinisches Bildgebungssystem nach Anspruch 8, wobei die Bildgebungseinheit eine Röntgenquelle (3) zum Aussenden einer Röntgenstrahlung und einen Röntgendetektor (4) zur Aufnahme von Bilddaten des durchstrahlten Untersuchungsobjekts (5) aufweist und die Steuerungseinheit (9) zur getriggerten Ansteuerung der Strahlungsausgabe und der Aufnahme von Bilddaten ausgebildet ist.

10. Medizinisches Bildgebungssystem nach Anspruch 9, wobei die Steuerungseinheit (9) derart ausgebildet ist, dass sie die Bestrahlung des Untersuchungsobjekts (5) und die Aufnahme der Bilddaten getriggert in Abhängigkeit von den ausgewerteten Messwerten steuert.

11. Medizinisches Bildgebungssystem nach einem der Ansprüche 8 bis 10, aufweisend eine Bildbearbeitungseinheit (11) zur Bearbeitung der Bilddaten unter Verwendung der ausgewerteten Messwerte.

12. Medizinisches Bildgebungssystem nach einem der Ansprüche 9 bis 11, aufweisend eine Registrierungseinheit zur Durchführung einer 2D-2D-3D-Registrierung zwischen zwei aus verschiedenen Projektionsrichtungen zum selben Zeitpunkt und/oder derselben Phase der zyklischen Bewegung des Untersuchungsobjekts aufgenommenen (2D-)Projektionsaufnahmen und einem 3D-Volumenbild des Untersuchungsobjekts.

13. Medizinisches Bildgebungssystem nach einem der Ansprüche 8 bis 12, wobei das robotische Gerät an einem Ende einen Endeffektor aufweist, welcher Endeffektor dazu ausgebildet ist, mit dem Patienten taktil in Kontakt zu stehen.

14. Medizinisches Bildgebungssystem nach Anspruch 13, wobei der Endeffektor eine gezahnte Hülse aufweist.

## Claims

1. Method for recording image data of a moving, in particular cyclically moving, examination object (5) of a patient by means of a medical imaging system (1), wherein a robotic device (6) with a kinematic chain of moving components (7) has a tactile connection with the patient in that an end effector lies on the surface of the patient, and wherein the tactile connection is maintained at least for the duration of the method by means of force and/or torque regulation by way of conjoint movement of the moving components (7), and wherein meanwhile the following steps are performed:
• acquisition of measured values by a force and/or torque and/or position sensor system of the robotic device (6),
• evaluation of the measured values and forwarding to the medical imaging system (1), wherein the evaluated measured values in particular comprise information on the movement and/or position of the examination object (5), and
• recording of image data from the examination object (5) by the imaging system (1),
wherein the evaluated measured values are used to actuate the imaging system (1).

2. Method according to claim 1, wherein the medical imaging system (1) comprises an X-ray source (3) and an X-ray detector (4) and the recording of image data includes the irradiation of the examination object (5) by the X-ray source (3) and the recording of the irradiated examination object (5) by the X-ray detector (4).

3. Method according to claim 2, wherein the irradiation of the examination object (5) and the recording of the image data is controlled in a gated manner in dependence on the evaluated measured values, in particular by movement and/or positional data from the examination object (5).

4. Method according to claim 2 or 3, wherein at least one projection recording of the examination object (5) is recorded at a predetermined time and/or a predetermined phase of the in particular cyclic movement of the examination object (5).

5. Method according to claim 2 or 3, wherein at least two projection recordings of the examination object (5) are recorded at the same time and/or same phase of the in particular cyclic movement of the examination object (5).

6. Method according to claim 5, wherein the two projection recordings are recorded from different projection directions and used for 2D-2D-3D registration.

7. Method according to one of the preceding claims, wherein the recorded image data is processed using the evaluated measured values.

8. Medical imaging system (1) for the acquisition of image data of a cyclically moving examination object (5) of a patient, embodied for carrying out a method for recording image data of a cyclically moving examination object, having
• an imaging unit (3; 4) for recording image data from the examination object (5),
• a control unit (9) for the gated actuation of the recording of image data,
• a robotic device (6) with a kinematic chain of moving components (7) comprising a force and/or torque and/or position sensor system for recording measured values, which in particular comprise information on the movement and/or position of the examination object, wherein the robotic device (6) has a tactile connection with the patient and is embodied to maintain the tactile connection for the duration of the method by means of force and/or torque regulation by way of conjoint movement of the movable components (7),
• an evaluation unit (14) for the evaluation of the measured values and
• a communication link (12) for forwarding the measured values,
wherein the medical imaging system (1) is embodied to use the evaluated measured values to actuate the imaging system (1).

9. Medical imaging system according to claim 8, wherein the imaging unit comprises an X-ray source (3) for emitting X-rays and an X-ray detector (4) for recording image data of the irradiated examination object (5) and the control unit (9) is embodied for the gated actuation of the radiation output and the recording of image data.

10. Medical imaging system according to claim 9, wherein the control unit (9) is embodied such that it controls the irradiation of the examination object (5) and the recording of the image data gated in dependence on the evaluated measured values.

11. Medical imaging system according to one of claims 8 to 10 comprising an image processing unit (11) for processing the image data using the evaluated measured values.

12. Medical imaging system according to one of claims 9 to 11 comprising a registration unit for performing 2D-2D-3D registration between two (2D) projection recordings recorded from different projection directions at the same time and/or the same phase of the cyclic movement of the examination object and a 3D volume image of the examination object.

13. Medical imaging system according to one of claims 8 to 12, wherein the robotic device comprises an end effector at one end, which end effector is embodied to be in tactile contact with the patient.

14. Medical imaging system according to claim 13, wherein the end effector comprises a toothed sleeve.

## Revendications

1. Procédé d'enregistrement de données d'image d'un objet (5) à examiner se déplaçant, notamment de manière cyclique, d'un patient au moyen d'un système (1) d'imagerie médicale, dans lequel un appareil (6) robotisé est en liaison tactile avec le patient par une chaîne cinématique d'éléments (7) mobiles, par le fait qu'un préhenseur s'applique à la surface du patient, et dans lequel on maintient la liaison tactile au moins pendant la durée du procédé au moyen d'une régulation de force et/ou de couple avec co-déplacement des éléments (7) mobiles et dans lequel, pendant cela, s'effectuent les stades suivants :
• relevé de valeurs de mesure par des capteurs de force et/ou de couple et/ou de capteurs de position de l'appareil (6) robotisé,
• exploitation des valeurs de mesure et retransmission au système (1) d'imagerie médicale, les valeurs de mesure exploitées donnant notamment des informations sur le déplacement et/ou la position de l'objet à examiner et
• enregistrement de données d'image de l'objet (5) à examiner par le système (1) d'imagerie,
dans lequel on utilise les valeurs de mesure exploitées pour une commande du système (1) d'imagerie.

2. Procédé suivant la revendication 1, dans lequel le système (1) d'imagerie médicale a une source (3) de rayons X et un détecteur (4) de rayons X et l'enregistrement de données d'image comprend une irradiation de l'objet (5) à examiner par la source (3) de rayons X et un enregistrement de l'objet (5) à examiner irradié par le détecteur (4) de rayons X.

3. Procédé suivant la revendication 2, **caractérisé en ce que** l'on commande l'irradiation de l'objet (5) à examiner et l'enregistrement des données d'image en les déclenchant en fonction des valeurs de mesure exploitées, notamment des données de déplacement et/ou de position de l'objet (5) à examiner.

4. Procédé suivant la revendication 2 ou 3, dans lequel on enregistre au moins un enregistrement de projection de l'objet (5) à examiner à un instant déterminé à l'avance et/ou à une phase déterminée à l'avance du déplacement, notamment cyclique, de l'objet (5) à examiner.

5. Procédé suivant la revendication 2 ou 3, dans lequel on enregistre au moins deux enregistrements de projection de l'objet (5) à examiner au même instant et/ou à la même phase du déplacement, notamment cyclique, de l'objet (5) à examiner.

6. Procédé suivant la revendication 5, dans lequel on enregistre les deux enregistrements de projection dans des directions de projection différentes et on les utilise pour un enregistrement 2D-2D-3D.

7. Procédé suivant l'une des revendications précédentes, dans lequel on effectue, en utilisant les valeurs de mesure exploitées, un traitement des données d'image enregistrées.

8. Système (1) d'imagerie médicale pour l'acquisition de données d'image d'un objet (5) à examiner se déplaçant cycliquement d'un patient, constitué pour effectuer un procédé d'enregistrement de données d'image de l'objet à examiner se déplaçant cycliquement, comportant
• une unité (3; 4) d'imagerie pour l'enregistrement de données d'image de l'objet (5) à examiner,
• une unité (9) de commande pour la commande de manière déclenchée de l'enregistrement de données d'image,
• un appareil (6) robotisé ayant une chaîne cinétique d'éléments (7) mobiles, comportant un capteur de force et/ou de couple et/ou de position pour le relevé de valeurs de mesure, qui donnent notamment des informations sur le déplacement et/ou la position de l'objet à examiner, l'appareil (6) robotique étant en liaison tactile avec le patient et étant constitué pour maintenir la liaison tactile pendant la durée du procédé au moyen de la régulation de force et/ou de couple par co-déplacement des éléments (7) mobiles,
• une unité (14) d'exploitation pour exploiter les valeurs de mesure et
• une liaison (12) de communication pour retransmettre les valeurs de mesure,
dans lequel le système (1) d'imagerie médicale est constitué pour une utilisation des valeurs de mesure exploitées pour une commande du système (1) d'imagerie médicale.

9. Système d'imagerie médicale suivant la revendication 8, dans lequel l'unité d'imagerie a une source (3) de rayons X pour émettre un rayonnement X et un détecteur (4) de rayons X pour enregistrer des données d'image de l'objet (6) à examiner irradié et l'unité (9) de commande est constituée pour la commande de manière déclenchée de l'émission de rayonnement et l'enregistrement de données d'image.

10. Système d'imagerie médicale suivant la revendication 9, dans lequel l'unité (9) de commande est constituée de manière à commander l'irradiation de l'objet (5) à examiner et l'enregistrement des données d'image de manière déclenchée en fonction des valeurs de mesure exploitées.

11. Système d'imagerie médicale suivant l'une des revendications 8 à 10, comportant une unité (11) de traitement d'image pour le traitement des données d'image en utilisant les valeurs de mesure exploitées.

12. Système d'imagerie médicale suivant l'une des revendications 9 à 11, comportant une unité d'enregistrement pour effectuer un enregistrement 2D-2D-3D entre deux enregistrements de projection (2D-) enregistrés dans des directions de projection différentes au même instant et/ou à la même phase du déplacement cyclique de l'objet à examiner et enregistrer une image en volume en 3D de l'objet à examiner.

13. Système d'imagerie médicale suivant l'une des revendications 8 à 12, dans lequel l'appareil robotisé a à une extrémité un préhenseur, lequel préhenseur est constitué pour être en contact tactile avec le patient.

14. Système d'imagerie médicale suivant la revendication 13, dans lequel le préhenseur a un manchon denté.
